(19) Europäisches Patentamt

European Patent Office

Office européen des brevets

(11) **EP 1 681 050 A1**

(12) **EUROPEAN PATENT APPLICATION**

(43) Date of publication:
19.07.2006 Bulletin 2006/29

(21) Application number: 06100300.0

(22) Date of filing: 12.01.2006

(51) Int Cl.:
*A61K 9/20* (2006.01)     *A61K 9/36* (2006.01)
*A61K 31/43* (2006.01)     *A61K 31/546* (2006.01)
*A61P 31/04* (2006.01)

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 13.01.2005 IN MU08802004

(71) Applicant: Strides Arcolab Limited
Navi Mumbai 400 703 (IN)

(72) Inventors:
• **Singh, Kamalinder Kaur**
400 049, Maharashtra (IN)
• **Dahiwal, Madhuri**
400 049, Maharashtra (IN)

(74) Representative: **Brand, Thomas Louis**
W.P. Thompson & Co.,
55 Drury Lane
London WC2B 5SQ (GB)

(54) **Dispersible sustained release pharmaceutical compositions**

(57)     Disclosed herein are pharmaceutical compositions, in particular, dispersible compositions comprising sustained release granules of at least one active pharmaceutical ingredient and at least one release retard hydrophobic polymer formulated with super disintegrant and lubricant, to achieve dispersible and sustained release effect.

EP 1 681 050 A1

**Description**

**Field of the invention:**

The present invention relates to pharmaceutical compositions, in particular, dispersible compositions comprising sustained release granules of at least one active pharmaceutical ingredient and at least one release retard hydrophobic polymer formulated with an effective disintegrant and lubricant, to achieve dispersible and sustained release effect.

**Background of the invention:**

Certain medical conditions for example chronic pain, infections etc, require the administration of a pharmaceutical in such a way that its release is sustained over an extended period of time. This is achieved by repeated administration of an immediate release formulation of the required drug at frequent pre determined intervals. In such cases, sustained release formulations are preferred to be administered, which release the drug at predetermined time intervals, thus eliminating the need of repeated administration.
Effervescent and water dispersible dosage forms are preferred as they are convenient to administer especially to the pediatric patients or those who face difficulty in swallowing solid dosage forms.
The Pharmaceutical Journal, March 12, 1983, p289-294, F. E. J. Sendall et al. discloses effervescent and water-dispersible tablets which are know well known. However, prior to this it was not suggested that granular sustained-release formulations could be presented successfully in the form of effervescent or water-dispersible tablets.
However, subsequently research work was carried out to get formulations in the form of dispersible tablet with a sustained release drug profile. The prior art for the same is mentioned below.
EP0313328 describes a granular sustained-release formulation of a pharmacologically active substance presented in the form of a tablet. The said tablet comprises a core containing active ingredient with one or more excipients which is further coated with water insoluble but water swellable acrylic polymer and a water soluble hydroxylated cellulose derivative, thus providing sustained-release profile on administration to patients. In the said formulation the mannitol is used as water dispersing agent as a result of which the tablet disperses in the mouth.
EP0052075 describes a granular delayed-release form of pharmaceutical active substances, which contains a granulated or crystalline pharmaceutical active substance coated with coating materials retarding the release of the active substances, these coating materials consist of a homogeneous mixture of a polyacrylate insoluble but dispersible in water and a cellulose ether insoluble but dispersible in water.
US4988679 discloses an orally ingestible liquid composition for suspending therein an orally administrable pharmaceutically active composition releasable over an extended period of time which comprises triglyceride of a medium chain length alkanoic acid or distilled acetylated monoglycerides, a liquid, high HLB polyglyceryl ester, and colloidal silicon dioxide together with a material soluble or dispersible therein and capable of being insolubilized by a pharmaceutically acceptable polyvalent cation and a solid pharmaceutically acceptable salt containing the cation required therefore. Sustained release compositions based thereon containing pharmaceutically active agents are also disclosed
US5780055 describes cushioning beads comprising microcrystalline cellulose and a disintegrant (preferably croscarmellose sodium). The cushioning beads are prepared by extrusion-spheronization, followed by freeze-drying technique. This patent has been disclosed water-dispersible tablets having high tensile strength, comprising the cushioning beads and biologically active ingredient-loaded beads, wherein optionally, the tablets can contain a viscosity enhancer in the form of separate beads, or as a component of the biologically active ingredient-loaded beads. All these beads are prepared by extrusion-spheronization technique followed by freeze drying technique. Further, all these beads are blended and compressed to obtain a water dispersible tablet. The above prior art is very difficult and tedious to practice, thus, leaving ample scope to carry out further investigations.
Hence, the present invention is aimed to provide a cost effective, simple and industrially viable process to prepare dispersible sustained release compositions which are suitable particularly to pediatric and geriatric patients. Further the present formulation may be dispersed in water prior to administration. On the other hand the present formulation comprises granules made by wet granulation which are not coated like those mentioned in prior art.

**Objectives of the present invention:**

An objective of the present invention is to ameliorate one or more of the problems associated with prior art.
Another objective of the present invention is to provide a dispersible sustained release tablet composition which disintegrate rapidly in water, and form a homogenous suspension which can be easily swallowed by children and the elderly, with minimal effect on the release properties of the biologically active ingredient.
A further object of the present invention is to provide sustained release drug granules which are able to sustain the release of the active ingredient over a period of 12 hrs in the gastro intestinal tract.

Still another objective of the invention is to reduce the frequency of administrations and to provide for convenience of administration to geriatric and pediatric patients.

**Summary of the invention:**

The present invention discloses pharmaceutical compositions, in particular, dispersible compositions comprising sustained release granules of at least one active pharmaceutical ingredient and at least one release retard hydrophobic polymer formulated with super disintegrant and lubricant, to achieve dispersible and sustained release effect. Further, being in the solid dosage form the antibiotics are more stable in the said formulation.

**Detailed description of the invention:**

The dispersible sustained release tablet composition of the present invention is suitable for oral administration to pediatric and geriatric patients as it is easy to swallow and also provides sustained release of the active pharmaceutical ingredient from the granules, thus reducing the frequency of administration.

According to the present invention, the dispersible sustained release tablet compositions comprises sustained release granules of at least one active pharmaceutical ingredient and at least one release retard hydrophobic polymer, a super disintegrant, lubricant and other optional conventional agents such as coloring agents, flavoring agents and sweetening agents.

The composition of the present invention comprises at least one antibiotic along with other inert pharmaceutical excipients. The antibiotics are selected from the group of β-lactams, cephalosporins and/or penicillins.

In the preferred embodiment, the antibiotic is amoxicillin, in the form of amoxicillin-trihydrate. In the present invention, the pediatric dose of 200mg of amoxicillin to an adult dose of up to 1000mg tablets were prepared to deliver in a sustained release tablet composition at an interval of about 12 hours.

In another preferred embodiment, the antibiotic is cefixime in the form of cefixime trihydrate suitably formulated to achieve the dispersible and sustained effect.

The release retarding polymers used in the said formulation may be a hydrophobic polymer is selected from group consisting of methyl cellulose, ethylcellulose, Carbomers Eudragits , Hydroxy propyl methyl cellulose, Hydroxypropyl cellulose or Hydroxyethyl cellulose. One preferred hydrophobic polymer is ethylcellulose.

The ratio of the active pharmaceutical ingredient and ethylcellulose in the sustained release granules are in the range of 1:3. The active : ethylcellulose ratio may be from about 1:0.5 to about 1:3, for example from 1:0.5 or 1:0.75 or 1:1 or 1:2 or 1:3, most preferably 1:0.75.

The super disintegrants used may be croscarmellose sodium, crospovidone, sodium starch glycolate, and starch. The amount of super disintegrant may be 15 to 25% of tablet weight. One preferred super disintegrant is sodium starch glycolate.

The lubricant is selected from the group of metallic silicates, metallic stearates or colloidal silicon dioxide (Aerosil), starch, talc, micronized amorphous silica or amorphous silica. A lubricant employed may preferably be Aerosil. The amount of lubricant may be 10 to 15% of tablet weight.

The tablet formulation may also include other conventional excipients such as coloring agents, flavouring agents and sweeteners.

Suitable coloring agents for use in the formulation preferably include erythrosine at an amount of 0.3 to 0.8% of tablet weight.

Suitable flavouring agents for use in the formulation include strawberry, tutti-fruti, preferably strawberry at an amount of 0.3 to 0.8% of tablet weight.

Suitable sweetening agents for use in the formulation include saccharin sodium at an amount of 1.0 to 3.0% of tablet weight.

The sustained release granules of active ingredient were prepared by the wet granulation technique.

Procedure for preparation of sustained release granules by wet granulation technique:

The active ingredient was mixed with ethylcellulose and coloring agent in geometric proportion. Mixing was continued further for 1 hour so as to ensure uniform mixing. To the uniform mixture obtained, distilled ethanol was added slowly, so as to form dough which was then passed through sieve no. 16 and the granules obtained were air-dried.

Procedure for preparation of the sustained release dispersible tablets:

The glidant, Aerosil was sieved through sieve no. 85 to break any lumps. The color, erythrosine, was also sieved through sieve no. 85. The glidant and the color were then mixed intimately and again sieved through sieve no. 85 so as to get a uniform distribution of the color in the glidant.

The flavor strawberry/tutti-fruti, was separately sieved through sieve no. 85 and was mixed with the color and glidant mixture.

The sweetener sodium saccharin was crushed in a mortar and passed through sieve no. 45 and was mixed evenly with the above mixture of color, flavor and glidant mixture.

To the above mixture of color, flavor, glidant and sweetener, the disintegrant was added and mixed thoroughly. The disintegrant used maybe crospovidone, croscarmellose sodium or starch, preferably sodium starch glycollate(Primojel).

To the excipient mixture prepared, the active ingredient granules were mixed in geometric proportion. Mixing was continued for further for 30-45 mins so as to ensure uniform distribution of the granules in the excipient mix.

Tablets were punched using single a concave or capsule shaped die punch. Thus, coloured, circular, biconvex or capsule shaped tablets with a sweet odor with a hardness of 5-7 kg/cm$^2$ and a friability value of 0.2% were obtained. The dispersible tablets disintegrated within less than 1 minute when brought in contact with water at room temperature.

**Disintegration time:**

The disintegration test was carried out according to I.P. The tablet was placed in a disintegration tester containing around 800ml of water at room temperature. The tablet disintegrated within less than 1 min (in 45 sec.)

The following examples are provided to further illustrate the invention with specific embodiments stipulated in the present invention. The scope of the present invention is not restricted to amoxicillin but is intended to include any other class of antibiotics.

**Example 1**

| Ingredients | gm/tablet |
|---|---|
| Amoxicillin trihydrate | 0.2295 |
| Ethyl Cellulose | 0.1722 |
| Primojel | 0.151 |
| Aerosil | 0.091 |
| Erythrosine | 0.002 |
| Strawberry flavor | 0.005 |
| Sodium saccharin | 0.018 |

**Example 2**

| Ingredients | gm/tablet |
|---|---|
| Amoxicillin trihydrate | 0.2639 |
| Ethyl Cellulose | 0.2639 |
| Primojel | 0.151 |
| Aerosil | 0.091 |
| Erythrosine | 0.002 |
| Strawberry flavor | 0.005 |
| Sodium saccharin | 0.018 |

**Example 3**

| Ingredients | gm/tablet |
|---|---|
| Amoxicillin trihydrate | 0.2984 |
| Ethyl Cellulose | 0.373 |
| Primojel | 0.151 |
| Aerosil | 0.091 |
| Erythrosine | 0.002 |

(continued)

| Ingredients | gm/tablet |
|---|---|
| Strawberry flavor | 0.005 |
| Sodium saccharin | 0.018 |

**Example 4**

| Ingredients | gm/tablet |
|---|---|
| Amoxicillin trihydrate | 0.396 |
| Ethyl Cellulose | 0.792 |
| Primojel | 0.151 |
| Aerosil | 0.091 |
| Erythrosine | 0.002 |
| Strawberry flavor | 0.005 |
| Sodium saccharin | 0.018 |

**Example 5**

| Ingredients | gm/tablet |
|---|---|
| Amoxicillin trihydrate | 0.2869 |
| Ethyl Cellulose | 0.2152 |
| Primojel | 0.151 |
| Aerosil | 0.091 |
| Erythrosine | 0.002 |
| Strawberry flavor | 0.005 |
| Sodium saccharin | 0.018 |

**Example 6**

| Ingredients | gm/tablet |
|---|---|
| Amoxicillin trihydrate | 0.5763 |
| Ethyl Cellulose | 0.4322 |
| Primojel | 0.151 |
| Aerosil | 0.091 |
| Erythrosine | 0.002 |
| Strawberry flavor | 0.005 |
| Sodium saccharin | 0.018 |

Examples for Cefixime Trihydrate dispersible sustained release tablets

**Example 7**

| Ingredients | gm/tablet |
|---|---|
| Cefixime trihydrate | 0.2238 |
| Ethyl Cellulose | 0.1119 |

(continued)

| Ingredients | gm/tablet |
| --- | --- |
| Primojel | 0.151 |
| Aerosil | 0.091 |
| Erythrosine | 0.002 |
| Strawberry flavor | 0.005 |
| Sodium saccharin | 0.018 |

**Example 8**

| Ingredients | gm/tablet |
| --- | --- |
| Cefixime trihydrate | 0.2238 |
| Ethyl Cellulose | 0.1678 |
| Primojel | 0.151 |
| Aerosil | 0.091 |
| Erythrosine | 0.002 |
| Strawberry flavor | 0.005 |
| Sodium saccharin | 0.018 |

**Example 9**

| Ingredients | gm/tablet |
| --- | --- |
| Cefixime trihydrate | 0.2238 |
| Ethyl Cellulose | 0.2238 |
| Primojel | 0.151 |
| Aerosil | 0.091 |
| Erythrosine | 0.002 |
| Strawberry flavor | 0.005 |
| Sodium saccharin | 0.018 |

**Example 10**

| Ingredients | gm/tablet |
| --- | --- |
| Cefixime trihydrate | 0.4476 |
| Ethyl Cellulose | 0.2239 |
| Primojel | 0.175 |
| Aerosil | 0.12 |
| Erythrosine | 0.002 |
| Strawberry flavor | 0.0075 |
| Sodium saccharin | 0.02 |

**Example 11**

| Ingredients | gm/tablet |
|---|---|
| Cefixime trihydrate | 0.4476 |
| Ethyl Cellulose | 0.3357 |
| Primojel | 0.175 |
| Aerosil | 0.12 |
| Erythrosine | 0.002 |
| Strawberry flavor | 0.0075 |
| Sodium saccharin | 0.02 |

**Example 12**

| Ingredients | gm/tablet |
|---|---|
| Cefixime trihydrate | 0.4476 |
| Ethyl Cellulose | 0.4476 |
| Primojel | 0.175 |
| Aerosil | 0.12 |
| Erythrosine | 0.002 |
| Strawberry flavor | 0.0075 |
| Sodium saccharin | 0.02 |

**Example 13**

| Ingredients | gm/tablet |
|---|---|
| Cefixime trihydrate | 0.4476 |
| Ethyl Cellulose | 0.2239 |
| Primojel | 0.2 |
| Aerosil | 0.12 |
| Erythrosine | 0.002 |
| Strawberry flavor | 0.0075 |
| Sodium saccharin | 0.02 |

**Example 14**

| Ingredients | gm/tablet |
|---|---|
| Cefixime trihydrate | 0.4476 |
| Ethyl Cellulose | 0.3357 |
| Primojel | 0.2 |
| Aerosil | 0.12 |
| Erythrosine | 0.002 |
| Strawberry flavor | 0.0075 |
| Sodium saccharin | 0.02 |

**Example 15**

| Ingredients | gm/tablet |
|---|---|
| Cefixime trihydrate | 0.4476 |
| Ethyl Cellulose | 0.4476 |
| Primojel | 0.2 |
| Aerosil | 0.12 |
| Erythrosine | 0.002 |
| Strawberry flavor | 0.0075 |
| Sodium saccharin | 0.02 |

Dissolution testing methods:

The release of amoxicillin from the tablets was determined using the Dissolution Rate Test Apparatus 1, official in USP 22, by pH change method.

Test specifications:

| Temperature | : Room temp |
|---|---|
| Dissolution medium | : pH 1.2 buffer for the first 2 hours, 900 ml. |
| | pH 7.2 buffer for the remaining period, 900 ml |
| Basket Speed | : 100 rpm |

Method:

Tablet was tied in a nylon cloth and kept in the basket which was then immersed in the dissolution medium. Aliquots of medium were removed after 30 mins, 1 h, 1½h, 2h, 3h, 4h, 5h, 6h, 7h, 8h, each aliquot being replaced simultaneously by an equal volume of medium to maintain constant volume. The amount of active substance was determined by UV spectrometry, at 227 nm for both the buffers.

**Dissolution release profile for the examples 1to 6:**

| Time in h | % active ingredient released | | | | | |
|---|---|---|---|---|---|---|
| | **Example 1** | **Example 2** | **Example 3** | **Example 4** | **Example 5** | **Example 6** |
| 0 | 0 | 0 | 0 | 0 | 0 | 0 |
| 0.5 | 18.09 | 17.03 | 17.91 | 19.52 | 13.70 | 17.37 |
| 1 | 24.19 | 24.74 | 25.29 | 26.34 | 21.36 | 24.15 |
| 2 | 37.11 | 39.58 | 39.13 | 39.50 | 33.85 | 33.97 |
| 3 | 47.84 | 52.82 | 51.35 | 50.50 | 44.85 | 40.90 |
| 4 | 51.57 | 54.36 | 55.97 | 53.89 | 48.74 | 41.94 |
| 5 | 54.44 | 58.60 | 58.64 | 56.63 | 51.52 | 43.21 |
| 6 | 56.55 | 57.91 | 62.55 | 59.84 | 50.92 | 43.97 |
| 7 | 58.69 | 59.40 | 62.92 | 62.35 | 52.66 | 45.07 |
| 8 | 59.79 | 60.77 | 65.25 | 63.93 | 55.00 | 46.42 |

**In vivo evaluation of test formulation**

A comparison of bioavailability and other pharmacokinetic parameters of amoxicillin after administration of sustained release dispersible tablets and that of a commercial preparation was taken up to ascertain in vivo performance of developed formulation and compare it with a conventional product. Rabbit was used as an animal model.

**Protocol:**

Three adult rabbits of either sex, weighing between 1.5 to 2.5 kg were taken for the study. All the three rabbits received both the formulations. On the first day of the trial, the rabbits were given the test formulation. After a wash-out period of 7 days, the rabbits were given the commercial formulation.

Before administering the dosage form, control blood samples of 1ml were obtained from the marginal ear vein of all the rabbits. At zero time, the formulation was administered. Blood samples (1ml each) were then obtained at time intervals 0.5, 1, 1.5, 2, 4, 6, 8 and 24 h after the administration of the dose. Each of the blood sample was collected in the test tube containing 0.3ml of 3.8% w/v sodium citrate. The blood samples were centrifuged within 30mins after collection, and the plasma was separated and stored at - 20°C until analysis. Amoxicillin was assayed by the agar well diffusion method.

Pharmacokinetic parameters of amoxicillin in rabbit after oral administration of Test and commercial formulation

| Formulation | Rabbit | $C_{max}$ ($\mu$g/ml) | $AUC_{0-24h}$ ($\mu$g.h/ml) | MRT (h) |
|---|---|---|---|---|
| Commercial Sample | 1. | 9.21 | 26.17 | 4.2 |
| | 2. | 8.79 | 24.83 | 4.4 |
| | 3. | 8.26 | 24.47 | 4.5 |
| | Mean $\pm$ S.D. | 8.75$\pm$ 0.38 | 25.16$\pm$ 0.73 | 4.4$\pm$ 0.12 |
| Test Formulation | 1. | 6.23 | 44.16 | 9.8 |
| | 2. | 5.25 | 44.01 | 10.3 |
| | 3. | 5.77 | 44.61 | 10.7 |
| | Mean $\pm$ S.D. | 5.75$\pm$ 0.4 | 44.30$\pm$ 0.25 | 10.25$\pm$ 0.34 |

Statistical analysis of the pharmacokinetic parameters calculated for the amoxicillin test formulation and commercial formulation

| Variable | Student's t -test | 5% significan ce level | Analysis of variance (ANOVA) | 5% significanc e level |
|---|---|---|---|---|
| Drug plasma conc ($\mu$g/ml) in commercial and test formulations | 2.21 | Significant | - | - |
| $AUC_{0-24h}$ ($\mu$g.h/ml) in commercial and test formulations | 6.07 | Significant | - | - |
| Drug plasma conc ($\mu$g/ml) in commercial formulation, within the 3 rabbits | - | - | 4.89 | Significant |
| Drug plasma conc ($\mu$g/ml) in test formulation, within the 3 rabbits | - | - | 2.24 | Not Significant |

(continued)

| Variable | Student's t -test | 5% significance level | Analysis of variance (ANOVA) | 5% significance level |
|---|---|---|---|---|
| $AUC_{0-24h}$ (μg.h/ml) in commercial formulation, within the 3 rabbits | - | - | 5.02 | Significant |
| $AUC_{0-24h}$ (μg.h/ml) in test formulation, within the 3 rabbits | - | - | 0.107 | Not Significant |

The drug from the test formulation of amoxicillin was absorbed rapidly within half-an-hour giving a peak concentration of 5.75μg/ml. Drug plasma concentration declined gradually with time. The concentration at the end of 2h was 2.09μg/ml and the levels were maintained between 1.87-1.8μg/ml from the 4th h till the 8th h. Significant levels of amoxicillin (1.43μg/ml) were present in plasma after 24h.

Analysis of variance (ANOVA) at 5% significance level, indicated no significant difference in the plasma concentration and the (AUC) within the rabbits.

As compared to test formulation, commercial formulation showed a mean peak concentration of 8.75μg/ml at half-an-hour post administration after which the levels declined rapidly. The concentration at the end of 2h was 2.62μg/ml and at the end of 8h a drug plasma concentration of 1.03μg/ml was obtained. Blood sample withdrawn at 24h showed no amoxicillin.

Student's t-test, at 5% significance level, indicated a significant difference in the plasma drug concentration and the AUC between the test and the commercial formulations.

The plasma concentration data of both the commercial and test a formulation was evaluated by statistical moment analysis in terms of mean residence time (MRT).

MRT is calculated as:

$$MRT \quad = \quad \frac{AUMC}{AUC}$$

where,
AUMC is the area under the 'first moment curve' and is obtained from a plot of the product of drug concentration in plasma and time v/s time.

AUC is the area under the 'zero moment curve' and is obtained by plotting the drug concentration in plasma v/s time.

It was observed that MRT of the test formulation was 10.25h and that of the commercial formulation was 4.4h. An increase of about 6h was observed in the MRT of the test formulation as compared to the commercial formulation indicating a sustained effect of the test formulation.

High plasma amoxicillin levels do not correspond to increased efficacy of the drug. Though low drug levels in blood seem to indicate poor bioavailability, this however has no relevance from the therapeutic point of view. The important factor is the time period over which the drug levels are maintained above the minimum inhibitory concentration (MIC).

The test formulation maintained the drug levels above the MIC for a longer period of time than the commercial formulation. MIC (0.25μg/ml) for the susceptible pathogen was maintained upto 24h by the test formulation as compared to the commercial formulation. Also the commercial formulation gave too high $C_{max}$ values which are not desirable and may lead to side effects of the drug.

It can thus be concluded that the developed tablet formulation maintained the drug levels for a longer period of time and had a mean residence time of 10.25h and thus showed a significant sustained action as compared to the commercial formulation.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

**Claims**

1. A dispersible sustained release tablet composition comprising sustained release granules of at least one active pharmaceutical ingredient and at least one release retard hydrophobic polymer, a super disintegrant and a lubricant.

2. The dispersible sustained release tablet composition as claimed in claim 1 wherein said release retard hydrophobic polymer is selected from methyl cellulose, ethylcellulose, carbomers, eudragits hydroxy propyl methyl cellulose, hydroxy propyl cellulose, hydroxy ethyl cellulose, ethyl cellulose, or mixtures of two or more thereof.

3. The dispersible sustained release tablet composition as claimed in claim 1 or claim 2, wherein the weight ratio in the tablet composition of said active pharmaceutical ingredient and the release retard polymer ratio is in the range of 1:0.5 to 1:3.

4. The dispersible sustained release tablet composition as claimed in claim 3, wherein said active pharmaceutical ingredient and the release retard polymer ratio are present in the tablet composition in the ratio of 1: 0.75.

5. The dispersible sustained release tablet composition as claimed in any preceding claim, wherein said granules are prepared by a wet granulation technique.

6. The dispersible sustained release tablet composition as claimed in any preceding claim, wherein said active pharmaceutical ingredient comprises an antibiotic.

7. The dispersible sustained release tablet composition as claimed in any of the preceding claims, wherein said active pharmaceutical ingredient is selected from the group of β-lactams, cephalosporins or penicillins.

8. The dispersible sustained release tablet composition as claimed in any of the preceding claims, wherein said super disintegrant is selected from croscarmellose sodium, crospovidone, primojel, sodium starch glycolate, starch, or mixtures of two or more thereof.

9. The dispersible sustained release tablet as claimed in any preceding claim wherein the super disintegrant is present in an amount of 15 to 25% of the tablet weight.

10. The dispersible sustained release tablet composition as claimed in any preceding claim, wherein said lubricant is selected from the group of metallic silicates, metallic stearates starch, talc, micronized amorphous silica, amorphous silica, colloidal silicon dioxide (Aerosil), or mixtures of two or more thereof.

11. The dispersible sustained release tablet composition as claimed in any preceding claim wherein the lubricant is present in an amount of 5-15% of the tablet weight.

12. The dispersible sustained release tablet composition as claimed in any preceding claim further comprises coloring agent in an amount of 0.3 to 0.8% of the tablet weight.

13. The dispersible sustained release tablet composition as claimed in any preceding claim, further comprises flavoring agent in an amount of 0.3 to 0.8% of the tablet weight.

14. The dispersible sustained release tablet composition as claimed in any preceding claim, further comprises suitable sweetening agents in an amount of 1.0 to 3.0% of tablet weight.

15. The dispersible sustained release tablet composition as claimed in any of the preceding claims wherein said granules are able to sustain the release of the active ingredient over a period of 12 hrs in gastro intestinal tract.

16. The dispersible sustained release tablet composition as claimed in any of the preceding claims wherein said active pharmaceutical ingredient is amoxicillin trihydrate.

17. The dispersible sustained release tablet composition as claimed in any of the preceding claims wherein said active pharmaceutical ingredient is cefixime trihydrate.

**European Patent Office**

**EUROPEAN SEARCH REPORT**

Application Number

EP 06 10 0300

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/047808 A (LEK PHARMACEUTICALS D.D; KERC, JANEZ; SALOBIR, MATEJA) 10 June 2004 (2004-06-10) <br> * page 2, line 9 - line 16 * <br> * page 4, paragraph 2 * <br> * page 10; example 1 * <br> ----- | 1,2,5-8, 10,11, 13,16 | A61K9/20 <br> A61K9/36 <br> A61K31/43 <br> A61K31/546 <br> A61P31/04 |
| X | US 2004/005361 A1 (KHANDELWAL SANJEEV ET AL) 8 January 2004 (2004-01-08) <br> * page 3; example 1 * <br> * page 4, paragraph 57 - paragraph 59 * <br> ----- | 1,2,5-8, 10,15,17 | |
| X | WO 2004/073695 A (LEK PHARMACEUTICALS D.D; KERC, JANEZ; OPARA, JERNEJA; JENKO OSEL, MAJA) 2 September 2004 (2004-09-02) <br> * page 2, paragraph 3 * <br> * page 21 - page 22; example 4 * <br> * claim 22 * <br> ----- | 1,2,6,7, 10,12, 15,16 | |
| X | WO 2004/019901 A (ORCHID CHEMICALS & PHARMACEUTICALS LTD; KSHIRSAGAR, RAJESH, SURESH; DE) 11 March 2004 (2004-03-11) <br> * page 19; example 1 * <br> * page 34, line 11 - line 14 * <br> ----- | 1,2,6-8, 10,15 | TECHNICAL FIELDS SEARCHED (IPC) <br> A61K |
| X | US 6 136 345 A (GRIMMETT ET AL) 24 October 2000 (2000-10-24) <br> * column 5 - column 6; example 1 * <br> ----- | 1,6,7,9, 10,16 | |
| X | US 5 837 292 A (DIJKGRAAF ET AL) 17 November 1998 (1998-11-17) <br> * column 4, line 45 - line 60 * <br> * column 6; example 15 * <br> ----- | 1,2,5-8, 14,16 | |
| X | EP 1 226 818 A (PFIZER PRODUCTS INC) 31 July 2002 (2002-07-31) <br> * page 5, paragraph 47 - page 6, paragraph 50 * <br> ----- | 1,3-5,8, 10,16 | |

-/--

The present search report has been drawn up for all claims

| Place of search | Date of completion of the search | Examiner |
|---|---|---|
| The Hague | 9 March 2006 | Schüle, S |

**European Patent Office**

**EUROPEAN SEARCH REPORT**

Application Number

EP 06 10 0300

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/232077 A1 (DAGGY BRUCE P ET AL) 18 December 2003 (2003-12-18) * page 2, paragraph 15 * * page 2, paragraph 42 * * examples 7,8 * ----- | 1-3,5, 8-10 | |
| X | FR 2 116 M (VITAMIX PHARMACEUTICALS) 5 February 1962 (1962-02-05) * page 1, column 2 * * page 2, paragraph 1 * ----- | 1,2,5,6, 8,10 | |
| X | US 5 650 169 A (CONTE ET AL) 22 July 1997 (1997-07-22) * column 13 - column 14; example 5 * ----- | 1-5,8-10 | |
| | | | **TECHNICAL FIELDS SEARCHED (IPC)** |

The present search report has been drawn up for all claims

| Place of search | Date of completion of the search | Examiner |
|---|---|---|
| The Hague | 9 March 2006 | Schüle, S |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
..............................................................................................
& : member of the same patent family, corresponding
  document

EPO FORM 1503 03.82 (P04C01)

EP 1 681 050 A1

## ANNEX TO THE EUROPEAN SEARCH REPORT
## ON EUROPEAN PATENT APPLICATION NO.

EP 06 10 0300

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2006

| Patent document cited in search report | | Publication date | Patent family member(s) | | Publication date |
|---|---|---|---|---|---|
| WO 2004047808 | A | 10-06-2004 | AU | 2003302381 A1 | 18-06-2004 |
| | | | EP | 1581191 A1 | 05-10-2005 |
| US 2004005361 | A1 | 08-01-2004 | NONE | | |
| WO 2004073695 | A | 02-09-2004 | BR | 0407438 A | 07-02-2006 |
| | | | CA | 2516327 A1 | 02-09-2004 |
| | | | EP | 1596841 A1 | 23-11-2005 |
| WO 2004019901 | A | 11-03-2004 | AU | 2003260803 A1 | 19-03-2004 |
| US 6136345 | A | 24-10-2000 | DE | 69525148 D1 | 14-03-2002 |
| | | | DE | 69525148 T2 | 05-09-2002 |
| | | | WO | 9528148 A1 | 26-10-1995 |
| | | | EP | 0752850 A1 | 15-01-1997 |
| | | | JP | 9511994 T | 02-12-1997 |
| | | | US | 6358528 B1 | 19-03-2002 |
| US 5837292 | A | 17-11-1998 | UA | 61917 C2 | 15-12-2003 |
| | | | ZA | 9705848 A | 12-04-1999 |
| EP 1226818 | A | 31-07-2002 | CA | 2369268 A1 | 26-07-2002 |
| | | | JP | 2002226364 A | 14-08-2002 |
| US 2003232077 | A1 | 18-12-2003 | US | 6607749 B1 | 19-08-2003 |
| FR 2116 | M | | NONE | | |
| US 5650169 | A | 22-07-1997 | NONE | | |

EPO FORM P0459

For more details about this annex : see Official Journal of the European Patent Office, No. 12/82

15